# EUROPEAN PATENT SPECIFICATION

(11) **EP 2 848 274 B1**
(45) Date of publication and mention of the grant of the patent: **21.12.2016**
(21) Application number: 14153122.8
(22) Date of filing: 29.01.2014
(51) Int. Cl.: A61M 11/00, A61M 15/00

(54) **Medicament storage module**
Arzneimittelaufbewahrungsmodul
Module de stockage de médicaments

(30) Priority: 13.09.2013 TW 102133093
(43) Date of publication of application: 18.03.2015
(73) Proprietor: Delta Electronics, Inc., Taoyuan Hsien 333 (TW)
(72) Inventor: Lee, Kuo-Liang, Taoyuan Hsien 320 (TW); Lee, Yu-Wei, Taoyuan Hsien 320 (TW)
(74) Representative: 2K Patentanwälte Blasberg Kewitz & Reichel

(56) References cited:
- US-A1- 2006 207 591
- US-A1- 2007 267 010
- US-A1- 2008 060 640
- US-A1- 2013 119 151

## Description

### FIELD OF THE INVENTION

The present invention relates to a medicament storage module, and more particularly to a medicament storage module that has reduced residual medicament liquid and is easily cleaned.

### BACKGROUND OF THE INVENTION

Recently, a nebulizer has been widely used for health care and beauty purposes. For example, the nebulizer may be used to administer medicament to treat or alleviate pain of the patient with a respiratory disease. In addition, the nebulizer may be used to administer medicament to be inhaled into the lungs of the patient in replace of oral administration. Consequently, the absorption efficiency and action efficiency of the medicament are enhanced and the occurrence of the side effect is reduced. Moreover, the nebulizer can be carried to immediately administer medicament and quickly generate the drug action to the patient with exacerbation of the obstructive respiratory tract. In other words, the use of the nebulizer can immediately cure or relieve the patient in the event of sudden onset.

Generally, the nebulizer may be used to nebulize or vaporize the liquid medicament into mist droplets. After these mist droplets are inhaled by the mouth of the patient, these mist droplets can reach bronchus and lungs of the patient for therapy and alleviation of the respiratory disease. According to the nebulizing mechanism, the nebulizers are classified into several types, e.g. a pneumatic jet nebulizer, an ultrasonic nebulizer and a mesh nebulizer. The nebulizing mechanism of the mesh nebulizer for nebulizing liquid medicament into mist droplets comprises a piezoelectric element, a nozzle plate and a corresponding micro pump.

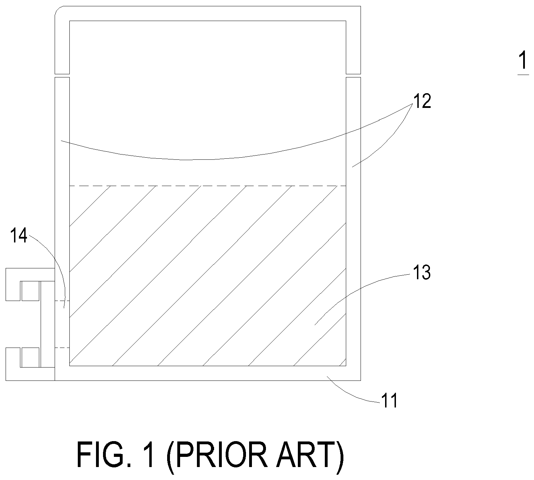

FIG. 1 is a schematic cross-sectional view illustrating a medicament cartridge of a conventional mesh nebulizer. As shown in FIG. 1, the medicament cartridge 1 is substantially a rectangular hollow structure. The medicament cartridge 1 comprises a bottom wall 11, four lateral walls 12, an accommodation space 13, and an outlet 14. The bottom wall 11 is a rectangular plate. The four lateral walls 12 are connected with each other and perpendicular to each other. Moreover, the four lateral walls 12 are perpendicularly connected with the edges of the bottom wall 11. The accommodation space 13 is defined by the bottom wall 11 and the four lateral walls 12 collaboratively. The outlet 14 runs through one of the four lateral walls 12, and is located near the bottom wall 11. The liquid medicament within the accommodation space 13 can be outputted or supplied through the outlet 14.

In the conventional medicament cartridge 1, the bottom wall 11 is in parallel with the horizontal plane, and the four lateral walls 12 are perpendicularly connected with the edges of the bottom wall 11. Consequently, a plurality of inside corners are formed at the bottom of the accommodation space 13. Due to the inside corners, a portion of the liquid medicament is usually retained at the bottom of the accommodation space 13 after the nebulizing operation of the nebulizer is completed. In other words, the conventional medicament cartridge 1 may result in the problems of wasting medicament or supplying insufficient amount of medicament. Moreover, since the medicament contained in the accommodation space 13 of the medicament cartridge 1 is viscous and the inside corners at the bottom of the accommodation space 13 are dead zones of liquid flow, the medicament is readily retained at the regions of the bottom wall 11 far from the outlet 14 and at the inside corners. Moreover, it is difficult to clean the conventional medicament cartridge 1. The subsequent use of the medicament cartridge 1 may contaminate the medicament or cause a bacteria problem.

Therefore, there is a need of providing a nebulizer and a medicament storage module in order to eliminate the above drawbacks.

US 2013 119 151 A relates to an aerosol generator assembly comprising a vibratable piezo ceramic body (2) having first and second opposing sides, an aperture defined in the vibratable body and extending through the body from the first side to the second side and having a layer of electrical contact material on each side of the vibratable body, the vibratable body being vibratable by application of an electrical signal thereto; a vibratable member (1) with pores defined therein, the vibratable member mounted across the aperture; and an electrical contact material free-zone (4) is provided on at least one side of the body about the aperture, characterised in that the electrical contact material free-zone and the vibratable member are dimensioned so that the vibratable member is mountable directly onto the vibratable body on top thereof within the electrical contact material free-zone such that a gap area free of electrode contact material is formed between the terminating edge of the electrical contact material and the periphery of the vibratable member.

US 2007 267 010 A relates to a method of treating a patient with a pulmonary disease, where the method includes delivering a dose of aerosolized medicament intermittently to a ventilator circuit coupled to the respiratory system of the patient. Also, a method of treating a patient with a pulmonary disease, where the method includes taking the patient off a ventilator, and administering to the patient, a nebulized aerosol comprising from about 100 mug to about 500 mg of a medicament.; Also a method of treating a patient with a pulmonary disease, the method comprising administering an aerosolized first medicament comprising amikacin to the patient and administering, systemically a second medicament comprising an antibiotic to the patient that also treats the pulmonary disease, wherein a resulting amikacin concentration in the lung and/or pulmonary system is therapeutically-effective, and an amount of the systemically administered antibiotics is reduced.

### SUMMARY OF THE INVENTION

The scope of the invention is as defined by the appended claims.

The present invention provides a medicament storage module for solving the problems of wasting medicament or supplying insufficient medicament.

The present invention provides a medicament storage module that is easily cleaned. In the subsequent use the problem of contaminating the medicament or causing bacteria growth will be avoided.

In accordance with an example there is provided a nebulizer. The nebulizer includes a medicament storage module and a nebulizing unit. The medicament storage module includes an inclined bottom wall, a tubular sidewall, and a guiding part. The inclined bottom wall is inclined at an inclination angle with respect to a horizontal plane. The tubular sidewall is connected with a periphery of the inclined bottom wall. A storage chamber with a top entrance is defined by the inclined bottom wall and the tubular sidewall collaboratively, and a liquid medicament is accommodated within the storage chamber. The tubular sidewall has an outlet, and the inclined bottom wall is extended downwardly to the outlet. The guiding part is arranged around a junction between the inclined bottom wall and the tubular sidewall. The nebulizing unit is disposed on the medicament storage module and aligned with the outlet. The liquid medicament outputted from the outlet is nebulized into a plurality of mist droplets by the nebulizing unit.

In accordance with the present invention, there is provided a medicament storage module. The medicament storage module includes an inclined bottom wall, a tubular sidewall, and a guiding part. The inclined bottom wall is inclined at an inclination angle with respect to a horizontal plane. The tubular sidewall is connected with a periphery of the inclined bottom wall. A storage chamber with a top entrance is defined by the inclined bottom wall and the tubular sidewall collaboratively, and a liquid medicament is accommodated within the storage chamber. The tubular sidewall has an outlet, and the inclined bottom wall is extended downwardly to the outlet. The guiding part is arranged around a junction between the inclined bottom wall and the tubular sidewall.

In accordance with a further aspect of the present invention, there is provided a medicament storage module. The medicament storage module includes an inclined bottom wall, a tubular sidewall, and a C-shaped curvy structure. The inclined bottom wall is inclined at an inclination angle with respect to a horizontal plane. The C-shaped curvy structure is arranged around a junction between the inclined bottom wall and the tubular sidewall. A storage chamber with a top entrance and an outlet is defined by the inclined bottom wall, the tubular sidewall and the C-shaped curvy structure collaboratively. A liquid medicament is accommodated within the storage chamber. The inclined bottom wall is extended downwardly to the outlet.

The above contents of the present invention will become more readily apparent to those ordinarily skilled in the art after reviewing the following detailed description and accompanying drawings, in which:

### BRIEF DESCRIPTION OF THE DRAWINGS

FIG. 1 is a schematic cross-sectional view illustrating a medicament cartridge of a conventional mesh nebulizer;
FIG. 2A is a schematic side view illustrating a nebulizer.
FIG 2B is a schematic cross-sectional view illustrating the nebulizer of FIG 2A and taken along the line AA;
FIG. 3A is a schematic perspective view illustrating the medicament storage module of the nebulizer of FIG. 2A and 2B;
FIG 3B is a schematic perspective view illustrating the medicament storage module of FIG 3A and taken along another viewpoint;
FIG. 3C is a schematic top view illustrating the medicament storage module of FIG. 3A;
FIG 3D is a schematic cross-sectional view illustrating the medicament storage module of FIG. 3A and taken along the line BB;
FIG. 4A is a schematic perspective view illustrating a variant example of the medicament storage module of the nebulizer wherein the upper cover is not shown;
FIG 4B is a schematic cross-sectional view illustrating the medicament storage module of FIG. 4A and taken along the line DD; and
FIG. 5 is a plot illustrating the relationship between the residual medicament percentage and the inclination angle of the inclined bottom wall of the medicament storage module of FIGS. 2A and 2B.

### DETAILED DESCRIPTION OF THE PREFERRED EMBODIMENT

The present invention will now be described more specifically with reference to the following embodiments. It is to be noted that the following descriptions of preferred embodiments of this invention are presented herein for purpose of illustration and description only. It is not intended to be exhaustive or to be limited to the precise form disclosed.

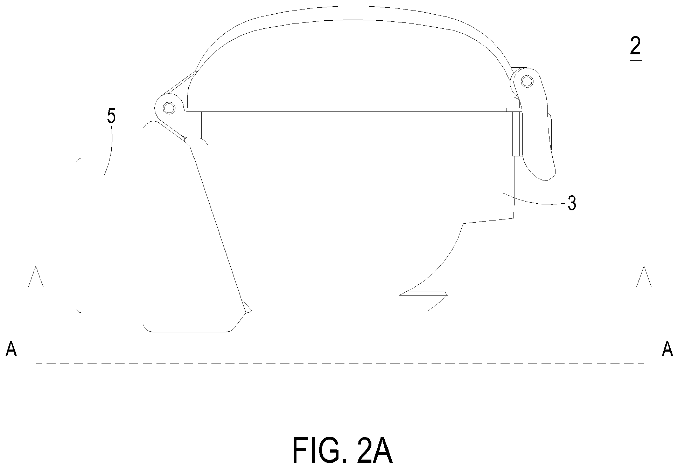

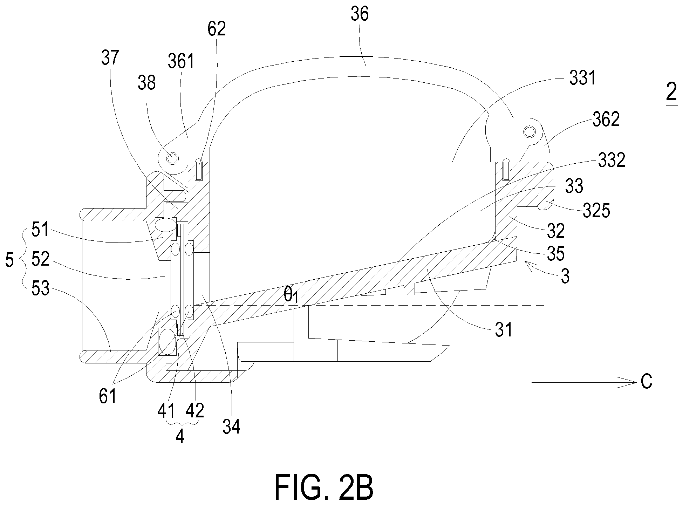

FIG. 2A is a schematic side view illustrating a nebulizer. FIG. 2B is a schematic cross-sectional view illustrating the nebulizer of FIG. 2A and taken along the line AA. An example of the nebulizer 2 includes but is not limited to a mesh nebulizer. The nebulizer 2 is used for nebulizing liquid medicament into a plurality of tiny mist droplets. For example, the mist droplets have a diameter in the range between 1 micrometer and 5 micrometers. After these mist droplets are inhaled by the mouth of the patient, these mist droplets can reach bronchus and lungs of the patient for therapy and alleviation of the respiratory disease.

In this example the nebulizer 2 comprises a medicament storage module 3, a nebulizing unit 4, and a conduit 5. The medicament storage module 3 is used for containing and supplying liquid medicament. The nebulizing unit 4 comprises a piezoelectric element 41 and a nozzle plate 42. The nebulizing unit 4 is disposed on the medicament storage module 3. By the nebulizing unit 4, the liquid medicament from the medicament storage module 3 is nebulized into the tiny mist droplets to be ejected. The conduit 5 is detachably connected with the medicament storage module 3, and aligned with the nebulizing unit 4. By the conduit 5, the tiny mist droplets ejected from the nebulizing unit 4 is guided to the mouth of the patient.

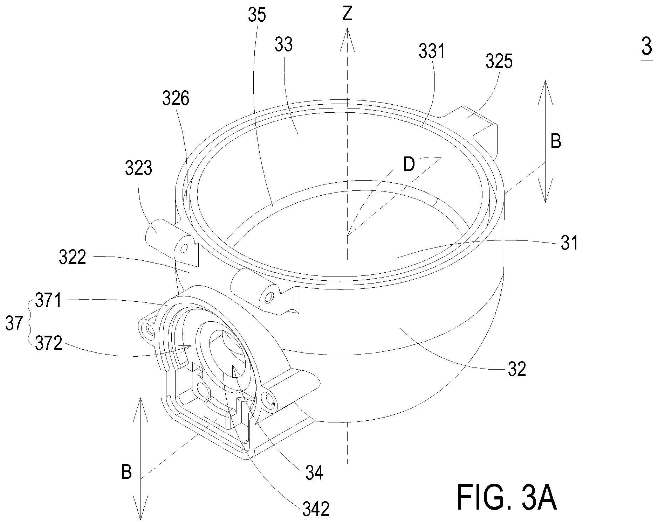

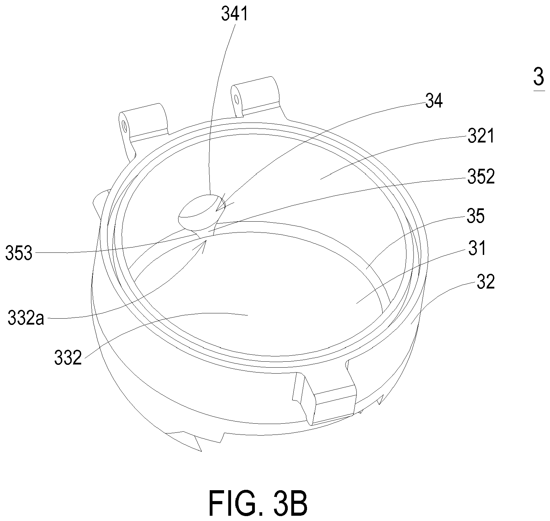

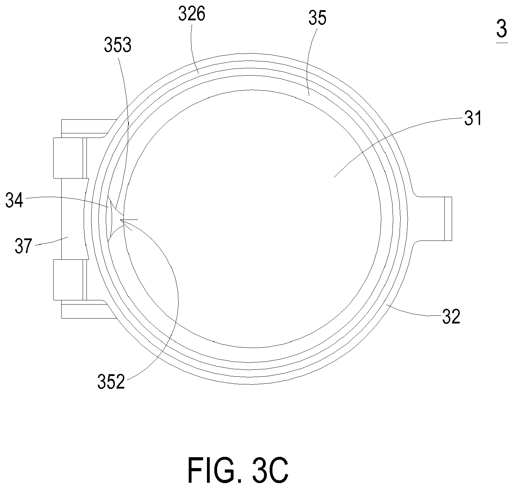

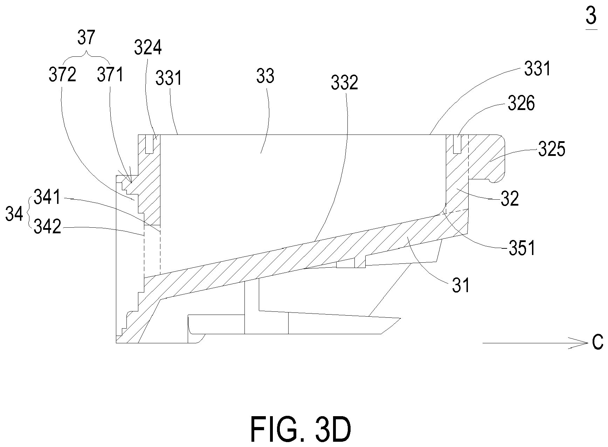

FIG. 3A is a schematic perspective view illustrating the medicament storage module of the nebulizer of FIG. 2A and 2B. FIG. 3B is a schematic perspective view illustrating the medicament storage module of FIG. 3A and taken along another viewpoint. FIG. 3C is a schematic top view illustrating the medicament storage module of FIG. 3A. FIG. 3D is a schematic cross-sectional view illustrating the medicament storage module of FIG. 3A and taken along the line BB. Please refer to FIGS. 2A, 2B, 3A, 3B, 3C and 3D. The medicament storage module 3 has a cup-shaped structure. In addition, the medicament storage module 3 comprises an inclined bottom wall 31, a tubular sidewall 32, a storage chamber 33, an outlet 34, and a guiding part 35. The inclined bottom wall 31 is inclined at an inclination angle θ1 with respect to a horizontal plane C. The inclination angle θ1 is in the range between 3 and 30 degrees, and preferably in the range between 5 and 15 degrees. The tubular sidewall 32 is connected with a periphery of the inclined bottom wall 31. Moreover, the storage chamber 33 has a top entrance 331 at a top side thereof. The storage chamber 33 is defined by the inclined bottom wall 31 and the tubular sidewall 32. The storage chamber 33 is used for accommodating or storing the liquid medicament. In an embodiment, the tubular sidewall 32 and the inclined bottom wall 31 are integrally formed with each other, and the tubular sidewall 32 is protruded upwardly from the periphery of the inclined bottom wall 31. Preferably, the tubular sidewall 32 is a circular sidewall. That is, the tubular sidewall 32 or the storage chamber 33 has a center axis Z. The center axis Z is perpendicular to the horizontal plane C. Moreover, there is an equidistance D between the center axis Z and the tubular sidewall 32. In this embodiment, the thickness and the diameter of the inclined bottom wall 31 and the thickness and the height of the tubular sidewall 32 may be varied according to the practical requirements.

The guiding part 35 of the medicament storage module 3 is located at a bottom surface 332 of the storage chamber 33, and arranged around the junction between the inclined bottom wall 31 and the tubular sidewall 32. In other words, the guiding part 35 is arranged around the periphery of the inner surface 332 of the inclined bottom wall 31 of the storage chamber 33. By means of the guiding part 35, no inside corners are formed within the storage chamber 33 of the medicament storage module 3. Consequently, the possibility of retaining the liquid medicament in the storage chamber 33 can be reduced, and the storage chamber 33 can be easily cleaned. In this embodiment, the guiding part 35 is a C-shaped curvy structure, but is not limited thereto. Preferably, the C-shaped curvy structure 35 has a concave curvy surface 351. Moreover, the radian of the curvy surface 351 of the C-shaped curvy structure 35 is not restricted, and may be varied according to the practical requirements. In some embodiments, the inclined bottom wall 31, the tubular sidewall 32 and the guiding part 35 are integrally formed with each other.

The outlet 34 is formed in the tubular sidewall 32 of the medicament storage module 3. That is, the outlet 34 runs through the tubular sidewall 32. The liquid medicament within the storage chamber 33 can be outputted or supplied through the outlet 34. The outlet 34 has a first opening 341 and a second opening 342. The first opening 341 is open to an inner surface 321 of the tubular sidewall 32 and aligned with a notch 352 of the guiding part 35. The second opening 342 is open to an outer surface 322 of the tubular sidewall 32. In this embodiment, the inclined bottom wall 31 is extended downwardly to the outlet 34 and aligned with the nebulizing unit 4. The first opening 341 of the outlet 34 is close to a lowest position 332a of the inner surface 332 of the inclined bottom wall 31 of the storage chamber 33 (see FIG. 3B). The distance between the lowest position 332a of the inner surface 332 of the inclined bottom wall 31 of the storage chamber 33 and the horizontal plane C is the shortest among all distances between the inner surface 332 of the inclined bottom wall 31 of the storage chamber 33 and the horizontal plane C. In an embodiment, the both sides of the notch 352 of the guiding part 35 have beveled edges 353 for facilitating guiding the liquid medicament to the outlet 34.

The medicament storage module 3 further comprises a coupling part 37. The coupling part 37 is connected with the outer surface 322 of the tubular sidewall 32 and aligned with the second opening 342 of the outlet 34. After the nebulizing unit 4 is installed in the coupling part 37, the nozzle plate 42 of the nebulizing unit 4 is aligned with the second opening 342 of the outlet 34. In an embodiment, the coupling part 37 and the tubular sidewall 32 are integrally formed with each other, and the coupling part 37 is extended externally from the outer surface 322 of the tubular sidewall 32 and aligned with the second opening 342 of the outlet 34. The coupling part 37 comprises an extension wall 371 and a first recess 372. The first recess 372 is defined by the extension wall 371 and the tubular sidewall 32 collaboratively. Moreover, the lateral surface of the first recess 372 has a multi-stepped structure mating with the piezoelectric element 41 and the nozzle plate 4 of the nebulizing unit 4, so that the nozzle plate 42 is aligned with the second opening 342 of the outlet 34.

The conduit 5 is detachably connected with the coupling part 37. In this embodiment, the conduit 5 comprises a coupling part 51, an entrance 52, and a tube part 53. The coupling part 51 of the conduit 5 is coupled with the extension wall 371 of the coupling part 37, and fixed on the coupling part 37. The entrance 52 of the conduit 5 is aligned with the nozzle plate 42 of the nebulizing unit 4. After the mist droplets are ejected from the nebulizing unit 4, the mist droplets are guided by the tube part 53 of the conduit 5. In some embodiments, one or more waterproof gaskets 61 (e.g. ring-shaped rubbery gaskets) are arranged between the nebulizing unit 4 and the outer surface 322 of the tubular sidewall 32 and between the nebulizing unit 4 and the coupling part 51 of the conduit 5 in order to prevent leakage of the liquid medicament.

The medicament storage module 3 further comprises an upper cover 36. The cover 36 is pivotally coupled to the tubular sidewall 32 for sheltering the top entrance 331 of the storage chamber 33. The tubular sidewall 32 further comprises at least one first shaft sleeve 323, and the cover 36 further comprises at least one second shaft sleeve 361. The medicament storage module 3 further comprises at least one shaft lever 38. After the shaft lever 38 is penetrated through the first shaft sleeve 323 and the second shaft sleeve 361, the cover 36 is pivotally coupled to the tubular sidewall 32. In a preferred embodiment, the first shaft sleeve 323 is integrally formed with the tubular sidewall 32, and located near a top edge 324 or the entrance 321 of the tubular sidewall 32, and located near the coupling part 37. Moreover, the tubular sidewall 32 further comprises at least one first engaging element 325, and the cover 36 comprises at least one second engaging element 362 corresponding to the at least one first engaging element 325. After the cover 36 is rotated to the position over the top entrance 331 of the storage chamber 33, the first engaging element 325 of the tubular sidewall 32 and the second engaging element 362 of the cover 36 are engaged with each other. Consequently, the top entrance 331 of the storage chamber 33 is tightly sealed by the cover 36. In an embodiment, the first engaging element 325 is located near the top edge 324 or the entrance 321 of the tubular sidewall 32, and is opposed to the first shaft sleeve 323. Moreover, a second recess 326 is formed in the top edge 324 of the tubular sidewall 32. A waterproof gasket 62 (e.g. a ring-shaped rubbery gasket) is accommodated within the second recess 326 in order to prevent leakage of the liquid medicament. In some other embodiments, the cover 36 further comprises one or more ventilation holes (not shown), but is not limited thereto.

The nebulizing unit 4 comprises the piezoelectric element 41 and the nozzle plate 42. The piezoelectric element 41 is a vibrating element for driving vibration of the nozzle plate 42. The nozzle plate 42 comprises a plurality of nozzles (not shown). As the nozzle plate 42 is vibrated by the piezoelectric element 41, the liquid medicament is nebulized into the tiny mist droplets to be ejected through the nozzles. The piezoelectric element 41 and the nozzle plate 4 of the nebulizing unit 4 are disposed within the first recess 372 of the coupling part 37 of the medicament storage module 3. In addition, the nozzle plate 42 is aligned with the second opening 342 of the outlet 34. Consequently, the liquid medicament outputted from the outlet 34 can be nebulized into the tiny mist droplets by the nebulizing unit 4. In an example the nozzle plate 42 has a circular profile. In particular, the nozzles of the nozzle plate 42 (i.e. the effective action region) are directly aligned with the second opening 342 of the outlet 34. Consequently, the nebulizing efficiency of the nebulizer 2 is enhanced.

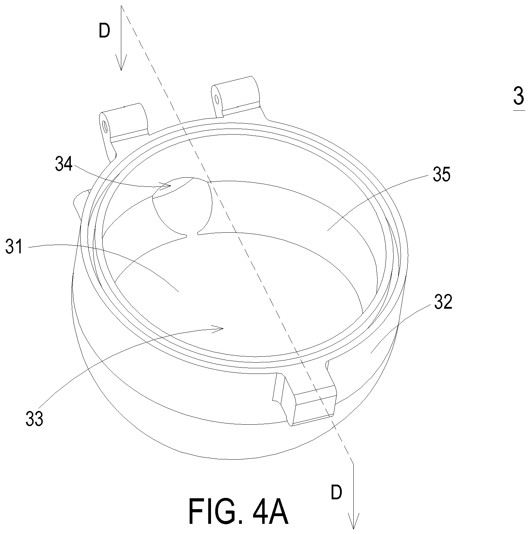

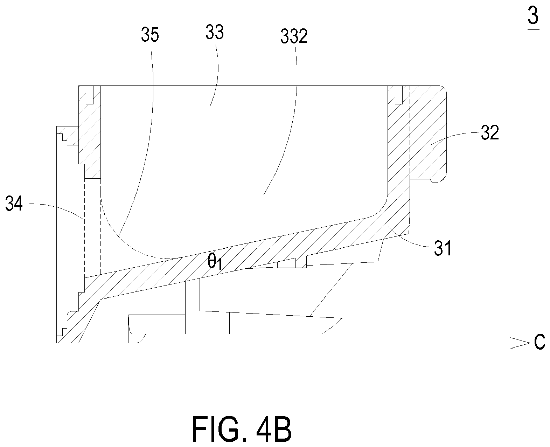

FIG. 4A is a schematic perspective view illustrating a variant example of the medicament storage module of the nebulizer of the present invention, wherein the upper cover is not shown. FIG. 4B is a schematic cross-sectional view illustrating the medicament storage module of FIG. 4A and taken along the line DD. As shown in FIGS. 4A and 4B, the medicament storage module 3 has a cup-shaped structure. In addition, the medicament storage module 3 comprises an inclined bottom wall 31, a tubular sidewall 32, a storage chamber 33, an outlet 34, and a C-shaped curvy structure 35. The inclined bottom wall 31 is inclined at an inclination angle θ1 with respect to a horizontal plane C. The inclination angle θ1 is in the range between 3 and 30 degrees, and preferably in the range between 5 and 15 degrees. The storage chamber 33 with a top entrance 331 and the outlet 34 is defined by the inclined bottom wall 31, the tubular sidewall 32 and the C-shaped curvy structure 33 collaboratively. The inclined bottom wall 31 is extended downwardly to the outlet 34. The C-shaped curvy structure 35 is connected with the inclined bottom wall 31 and tubular sidewall 32, and arranged around the periphery of the inner surface 332 of the inclined bottom wall 31 of the storage chamber 33. By means of the guiding part 35, no inside corners are formed within the storage chamber 33 of the medicament storage module 3. Consequently, the possibility of retaining the liquid medicament in the storage chamber 33 can be reduced, and the storage chamber 33 can be easily cleaned. The structures and functions of the inclined bottom wall 31, the tubular sidewall 32, the storage chamber 33, the outlet 34 and the C-shaped curvy structure 35 of this embodiment are similar to those illustrated in FIGS. 3A, 3B, 3C and 3D, and are not redundantly described herein.

In some examples the nebulizer 2 further comprises a handle (not shown), and the handle is detachably connected with the medicament storage module 3. Moreover, a power source (not shown) may be disposed within the handle for providing electric power to associated components of the nebulizer 2. Moreover, a control panel (not shown) may be disposed within the handle for controlling operations of the nebulizer 2.

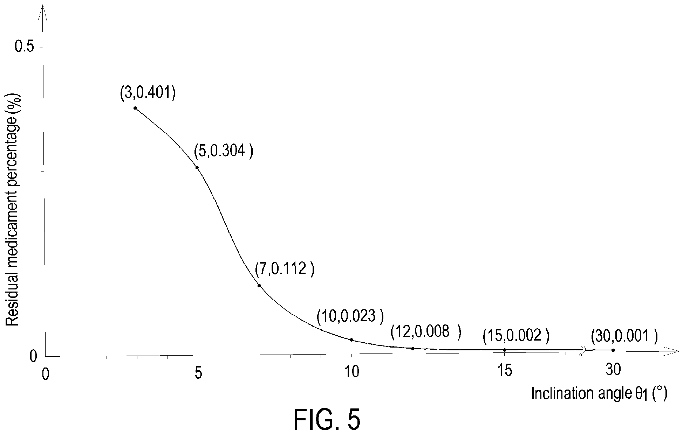

FIG. 5 is a plot illustrating the relationship between the residual medicament percentage and the inclination angle of the inclined bottom wall of the medicament storage module of FIGS. 2A and 2B. In case that the inclination angle θ1 of the inclined bottom wall 31 of the medicament storage module 3 is 3, 5, 7, 10, 12, 15 and 30 degrees, the residual medicament percentage is 0.401%, 0.304%, 0.112%, 0.023%, 0.008%, 0.002% and 0.001%, respectively. That is, if the inclination angle θ1 of the inclined bottom wall 31 in the range between 3 and 30 degrees, the residual medicament percentage of the nebulizer 2 can be effectively reduced. Especially, if the inclination angle θ1 of the inclined bottom wall 31 in the range between 5 and 15 degrees, the overall height and size of the nebulizer 2 and the storage chamber 33 of the medicament storage module 3 can be reduced while complying with the required medicament capacity (e.g. 4∼5ml). Consequently, the nebulizer 2 can be carried by the user more easily.

From the above descriptions, the present invention provides a medicament storage module. By means of a tubular sidewall and an inclined bottom wall with an inclination angle, the viscous liquid medicament can be easily guided to the outlet and outputted. By means of a guiding part (e.g. a C-shaped curvy structure) of the medicament storage module, no inside corners are formed within the storage chamber of the medicament storage module. Consequently, there are no dead zones in the storage chamber, and the liquid medicament can be naturally guided to the outlet by the guiding part. After the nebulizing operation of the nebulizer is completed, the residual medicament is reduced, and the problems of wasting medicament or supplying insufficient medicament can be overcome. Moreover, since the inclination angle is specially designed, the residual medicament percentage can be effectively reduced, and the overall height and size of the nebulizer and the storage chamber of the medicament storage module can be reduced while complying with the required medicament capacity (e.g. 4∼5ml). Consequently, the nebulizer can be carried by the user more easily. In the subsequent use of the nebulizer, the problem of contaminating the medicament or causing bacteria growth will be avoided. Moreover, the nebulizer of the preset invention can be used to administer medicament to treat or alleviate pain of the patient with a respiratory disease. In addition, the nebulizer can be used to administer medicament to be inhaled into the lungs of the patient in replace of oral administration. Consequently, the absorption efficiency and action efficiency of the medicament are enhanced and the occurrence of the side effect is reduced. Moreover, the nebulizer can be carried to immediately administer medicament and quickly generate the drug action to the patient with exacerbation of the obstructive respiratory tract. In other words, the use of the nebulizer can immediately cure or relieve the patient in the event of sudden onset.

## Claims

1. A medicament storage module (3) for nebulizers,
comprising:
an inclined bottom wall (31) disposed in the bottom and inclined at an inclination angle (θ1) with respect to a horizontal plane (C);
a tubular sidewall (32) preferably connected with a periphery of the inclined bottom wall (31); and
a guiding part (35) arranged around a junction between the inclined bottom wall (31) and the tubular sidewall (32);
**characterized in that** a storage chamber (33) with a top entrance (331) is defined by the inclined bottom wall (31) and the tubular sidewall (32) collaboratively, and a liquid medicament is accommodated within the storage chamber (33), wherein the tubular sidewall (32) has an outlet (34), the inclined bottom wall (31) is extended downwardly to the outlet (34), and the storage chamber (33) has a center axis Z, which is perpendicular to the horizontal plane C, and the top entrance (331) and the outlet (34) are axially perpendicular to each other, and the outlet (34) has a first opening (341) and a second opening (342), wherein the first opening (341) is open to an inner surface (321) of the tubular sidewall (32) and aligned with a notch (352) of the guiding part (35), and the second opening (342) is open to an outer surface (322) of the tubular sidewall (32).

2. The medicament storage module according to claim 1, wherein the inclination angle (θ1) is in a range between 3 and 30 degrees.

3. The medicament storage module according to claim 1, wherein the inclination angle (θ1) is in a range between 5 and 15 degrees.

4. The medicament storage module according to claim 1, wherein both sides of the notch (352) of the guiding part (35) have beveled edges (353).

5. The medicament storage module according to claim 1, wherein the guiding part (35) is a C-shaped curvy structure (35), the C-shaped curvy structure (35) is connected with the inclined bottom wall (31) and tubular sidewall (32), and arranged around the periphery of a inner surface (332) of the inclined bottom wall (31).

6. The medicament storage module according to claim 1, wherein the outlet (34) is adjacent to a lowest position (332a) of inner surface (332) of the inclined bottom wall (31).

7. The medicament storage module according to claim 1, wherein the medicament storage module (3) further comprises a cover (36), wherein the cover (36) is pivotally coupled to the tubular sidewall (32) for sheltering the top entrance (331) of the storage chamber (33).

8. The medicament storage module according to claim 7, wherein the tubular sidewall (32) further comprises at least one first engaging element (325), and the cover (36) comprises at least one second engaging element (362) corresponding to the at least one first engaging element (325), when the cover is closed, the at least one first engaging element (325) and the at least one second engaging element (362) are engaged with each other.

9. The medicament storage module according to claim 1, wherein a nebulizing unit (4) is disposed on the medicament storage module (3) and aligned with the outlet (34), wherein in use the liquid medicament outputted from the outlet (34) is nebulized into a plurality of mist droplets by the nebulizing unit (4), wherein the medicament storage module (3) further comprises a coupling part (37), and the coupling part (37) is connected with an outer surface (322) of the tubular sidewall (32) and aligned with the outlet (34), wherein the coupling part (37) comprises an extension wall (371) and a first recess (372), and the nebulizing unit (4) is disposed within the first recess (372).

10. The medicament storage module to claim 9, further comprising a conduit (5), wherein the conduit (5) is detachably connected with the coupling part (37) of the medicament storage module (3).

11. The medicament storage module to claim 1, wherein a second recess (326) is formed in a top edge (324) of the tubular sidewall (32), and a waterproof gasket (62) is accommodated within the second recess (326).

12. The medicament storage module to claim 1, wherein the nebulizing unit (4) is disposed on the medicament storage module (3) and aligned with the outlet (34), wherein in use the liquid medicament outputted from the outlet (34) is nebulized into a plurality of mist droplets by the nebulizing unit (4), wherein the nebulizing unit (4) comprises a piezoelectric element (41) and a nozzle plate (42).

## Patentansprüche

1. Ein Medikamenten- Speichermodul (3) für Zerstäuber, umfassend:
eine geneigte Bodenwand (31), die in dem Boden angeordnet ist und die geneigt ist mit einem Neigungswinkel (θ1) in Bezug auf eine horizontale Ebene (C);
eine rohrförmige Seitenwand (32), die vorzugsweise mit einem Randbereich der geneigten Bodenwand (31) verbunden ist; und
ein Führungsteil (35), das um eine Verbindungsstelle zwischen der geneigten Bodenwand (31) und der rohrförmigen Seitenwand (32) angeordnet ist;
**dadurch gekennzeichnet, dass** eine Aufbewahrungskammer (33) mit einem oberen Eingang (331) durch die geneigte Bodenwand (31) und die rohrförmige Seitenwand (32) gemeinsam bestimmt ist und ein flüssiges Medikament ist in der Speicherkammer (33) untergebrach, wobei die rohrförmige Seitenwand (32) einen Auslass (34) aufweist, die geneigte Bodenwand (31) erstreckt sich absteigend zum Auslass (34), und die Speicherkammer (33) weist eine Mittelachse Z auf, die senkrecht zu der horizontalen Ebene C ist, und der obere Eingang (331) und der Auslass (34) sind axial senkrecht zueinander, und der Auslass (34) weist eine erste Öffnung (341) und eine zweite Öffnung (342) auf, wobei die erste Öffnung (341) zu einer Innenfläche (321) der rohrförmigen Seitenwand (32) hin offen ist und mit einer Aussparung (352) des Führungsteils (35) verbunden ist und die zweite Öffnung (342) ist zu einer Außenfläche (322) der rohrförmigen Seitenwand (32) hin offen.

2. Das Medikamenten- Speichermodul nach Anspruch 1, wobei der Neigungswinkel (θ1) in einem Bereich zwischen 3 und 30 Grad liegt.

3. Das Medikamenten- Speichermodul nach Anspruch 1, wobei der Neigungswinkel (θ1) in einem Bereich zwischen 5 und 15 Grad liegt.

4. Das Medikamenten- Speichermodul nach Anspruch 1,wobei beide Seiten der Aussparung (352) des Führungsteils (35) abgeschrägte Kanten (353) aufweisen.

5. Das Medikamenten- Speichermodul nach Anspruch 1, wobei das Führungsteil (35) eine C- förmige, kurvige Struktur (35) hat, die C- förmige, kurvige Struktur (35) ist mit der geneigten Bodenwand (31) und der rohrförmigen Bodenwand (32) verbunden und ist um den Randbereich einer Innenfläche (332) der geneigten Bodenwand (31) angeordnet.

6. Das Medikamenten- Speichermodul nach Anspruch 1, wobei der Auslass (34) an eine unterste Position (332a) der Innenfläche (332) der geneigten Bodenwand (31) angrenzt.

7. Das Medikamenten- Speichermodul nach Anspruch 1, wobei das Medikamenten-Speichermodul (3) weiterhin eine Abdeckung (36) umfasst, wobei die Abdeckung (36) schwenkbar mit der rohrförmigen Seitenwand (32) zur Abdeckung des oberen Eingangs (331) der Aufbewahrungskammer (33) verbunden ist.

8. Das Medikamenten- Speichermodul nach Anspruch 7, wobei die rohrförmige Seitenwand (32) ferner mindestens ein erstes Eingriffselement (325) umfasst und die Abdeckung (36) umfasst mindestens ein zweites Eingriffselement (362), entsprechend zu dem mindestens einen ersten Eingriffselement (325), wenn die Abdeckung geschlossen ist, das mindestens eine erste Eingriffselement (325) und das mindestens eine zweite Eingriffselement (362) stehen miteinander in Eingriff.

9. Das Medikamenten- Speichermodul nach Anspruch 1,
wobei eine Zerstäubungseinheit (4) an dem Medikamenten- Speichermodul (3) angeordnet ist und mit dem Auslass (34) verbunden ist, wobei in Benutzung, das flüssige Medikament das von dem Auslass (34) ausgegeben wird, in eine Vielzahl von Nebeltröpfchen durch die Zerstäubungseinheit (4) zerstäubt wird,
wobei das Medikamenten- Speichermodul (3) ferner ein Kupplungsteil (37) aufweist und das Kupplungsteil (37) mit einer Außenfläche (322) der rohrförmigen Seitenwand (32) und mit dem Auslass (34) verbunden ist, wobei das Kupplungsteil (37) eine Verlängerungswand (371) und eine erste Ausnehmung (372) umfasst und die Zerstäubungseinheit (4) ist innerhalb der ersten Ausnehmung (372) angeordnet.

10. Das Medikamenten- Speichermodul nach Anspruch 9, ferner umfassend einen Kabelkanal (5), wobei der Kabelkanal (5) lösbar mit dem Kupplungsteil (37) des Medikamenten- Speichermoduls (3) verbunden ist.

11. Das Medikamenten- Speichermodul nach Anspruch 1, wobei eine zweite Ausnehmung (326) in einer oberen Kante (324) der rohrförmigen Seitenwand (32) ausgebildet ist und eine wasserundurchlässige Dichtung (62) ist in der zweiten Ausnehmung (326) untergebracht.

12. Das Medikamenten- Speichermodul nach Anspruch 1, wobei die Zerstäubungseinheit (4) an dem Medikamenten- Speichermodul (3) angeordnet ist und mit dem Auslass (34) verbunden ist, wobei in Benutzung das flüssige Medikament, das von dem Auslass (34) ausgegeben wird, in eine Vielzahl von Nebeltröpfchen durch die Zerstäubungseinheit (4) zerstäubt wird, wobei die Zerstäubungseinheit (4) ein piezoelektrisches Element (41) und eine Düsenplatte (42) umfasst.

## Revendications

1. Un module de stockage de médicament pour nébuliseurs, comprenant :
une paroi de fond inclinée (31) disposée au fond et inclinée selon un angle d'inclinaison (θ1) par rapport à un plan horizontal (C);
une paroi latérale tubulaire (32), de préférence raccordée à une périphérie de la paroi inférieure inclinée (31), et
un élément de guidage (35) disposé autour d'une jonction entre la paroi de fond inclinée (31) et la paroi latérale tubulaire (32) ;
**caractérisé en ce qu'**une chambre de stockage (33) ayant une entrée supérieure (331) est définie conjointement par la paroi de fond inclinée (31) et la paroi latérale tubulaire (32), et un médicament liquide est logé au sein de la chambre de stockage (33), dans lequel la paroi latérale tubulaire (32) comporte une sortie (34) et la paroi de fond inclinée (31) se prolonge vers le bas jusqu'à la sortie (34); et la chambre de stockage (33) présente un axe central Z, qui est perpendiculaire au plan horizontal C, et l'entrée supérieure (331) et la sortie (34) sont axialement perpendiculaire l'une vis à vis de l'autre, et la sortie (34) a une première ouverture (341) et une seconde ouverture (342), dans lequel la première ouverture (341) est ouverte sur une surface intérieure (321) de la paroi latérale tubulaire (32) et alignée avec une encoche de la partie de guidage (35), et la seconde ouverture (342) est ouverte sur une surface extérieure (322) de la paroi tubulaire (32).

2. Le module de stockage de médicament selon la revendication 1, dans lequel l'angle d'inclinaison (θ1) se situe dans une plage comprise entre 3 et 30 degrés.

3. Le module de stockage de médicament selon les revendications 1 ou 2, dans lequel l'angle d'inclinaison (θ1) se situe dans une plage comprise entre 5 et 15 degrés.

4. Le module de stockage de médicament selon la revendication 1, dans lequel les deux côtés de l'encoche (352) de la partie de guidage (35) ont des bords biseautés (353).

5. Le module de stockage de médicament selon la revendication 1 , dans lequel la partie de guidage (35) est une structure sinueuse en forme de C, la structure sinueuse en forme de C (35) étant raccordée à la paroi de fond inclinée (31) et à la paroi latérale tubulaire (32), et disposée autour de la périphérie d'une surface interne (332) de la partie de fond inclinée (31).

6. Le module de stockage de médicament selon la revendication 1, dans lequel la sortie (34) est proche d'une position la plus basse (332a) d'une surface inférieure (332) de la paroi de fond inclinée (31).

7. Le module de stockage de médicament selon la revendication 1, dans lequel le module de stockage de médicament (3) comporte en outre un couvercle (36), dans lequel le couvercle (36) est couplé de manière pivotante à la paroi latérale tubulaire (32) pour abriter l'entrée supérieure (331) de la chambre de stockage (33).

8. Le module de stockage de médicament selon la revendication 7, dans lequel la paroi latérale tubulaire (32) comprend en outre au moins un premier élément de prise (325) et le couvercle (36) comprend au moins un second élément de prise (362) correspondant au premier élément de prise (325) au moins lorsque le couvercle est fermé, dans lequel le premier élément de prise au moins (325) et le second élément de prise au moins (362) étant en prise l'un avec l'autre.

9. Le module de stockage de médicament selon la revendication 1, dans lequel une unité de nébulisation (4) est disposée sur le module de stockage de médicament et alignée avec la sortie (34), dans lequel, lors de l'utilisation, le médicament liquide produit par la sortie (34) est mise en nébulisation par l'unité de nébulisation (4) en une pluralité de gouttelettes, dans lequel le module de stockage de médicament (3) comporte en outre une pièce de couplage (37) et la pièce de couplage (37) est reliée à une surface extérieur (322) de la paroi latérale tubulaire (32) et alignée avec la sortie (34), dans lequel la pièce de couplage (37) comporte une paroi de prolongement (371) et un premier évidement (372) et l'unité de nébulisation (4) est disposée au sein du premier évidement (372).

10. Le module de stockage de médicament de la revendication 9, comprenant en outre un conduit (5), dans lequel le conduit (5) est raccordé de manière amovible à la pièce de couplage (37) du module de stockage de médicament.

11. Le module de stockage de médicament de la revendication 1, dans lequel un second évidement (326) est formé dans un bord supérieur (324) de la paroi latérale tubulaire (32), et un joint d'étanchéité (62) est logé dans le deuxième évidement (326).

12. Le module de stockage de médicament de la revendication 1, dans lequel l'unité de nébulisation (4) est disposée sur le module de stockage de médicament (3) et aligné avec la sortie (34), dans lequel, lors de l'utilisation, le médicament liquide produit par la sortie (34) est mise en nébulisation par l'unité de nébulisation (4) en une pluralité de gouttelettes, dans lequel l'unité de nébulisation (4) comporte un élément piézoélectrique (41) et une plaque de jet (42).
